# EUROPEAN PATENT SPECIFICATION

(11) **EP 0 384 976 B1**
(45) Date of publication and mention of the grant of the patent: **03.04.1996**
(21) Application number: 89123490.8
(22) Date of filing: 19.12.1989
(51) Int. Cl.: C11D 10/04, A61K 47/24, A23J 7/00

(54) **Glycerophospholipid composition having enhanced surface-active properties**
Glycerophospholipidzubereitung mit erhöhter Netzmittelwirkung
Composition à base de glycérophospholipides ayant des propriétés tensio-actives modifiées

(30) Priority: 23.12.1988 JP 325589/88
(43) Date of publication of application: 05.09.1990
(73) Proprietor: ASAHI DENKA KOGYO KABUSHIKI KAISHA, Arakawa-ku Tokyo 116 (JP)
(72) Inventor: Fujita, Satoshi c/o Asahi Denka Kogyo K.K., Tokyo (JP)
(74) Representative: Grünecker, Kinkeldey, Stockmair & Schwanhäusser Anwaltssozietät

(56) References cited:
- EP-A- 0 221 656
- WO-A-86/05694
- WO-A-89/10186
- GB-A- 2 113 568

## Description

This invention relates to a glycerophospholipid composition having enhanced surface-active properties. More particularly, it relates to a glycerophospholipid composition which is highly safe and has excellent surface-active properties including permeability, wettability and spreadability.

Examples of highly safe natural surfactants which are widely employed now in the fields of, for example, food, cosmetics, toiletry goods, drugs and agricultural chemicals include glycerophospholipids (phosphatides) such as soybean lecithin, which may be fractionated or partially treated with an enzyme so as to give lysophospholipids, and fatty acid monoglycerides. Among these surfactants, phosphatides containing a large amount of monoacyl(lyso)phosphatides are highly hydrophilic and show surface-active properties including permeation, wetting, spreading, dispersion and water-in-oil type emulsification. However, lysophosphatides such as lysophosphatidylcholine originating from soybean or yolk are considerably inferior in wettability and permeability to industrial surfactants such as polyoxyethylene nonylphenol ether or sodium dialkyl sulfosuccinates.

In addition to the above-mentioned fractionated or enzymatically treated products of glycerophospholipids, there have been attempted to blend glycerophospholipids with other lipids in order to elevate the emulsifying capability thereof. For example, Japanese Patent Publication No. 51241/1984, U.S. Patent No. 3,388,999, Danish Patent No. 101,694, U.S. Patent No. 3,661,795 and U.S. Patent No. 3,549,382 teach various surface-active compositions. However none of these compositions exerts a satisfactory effect.

Further, Japanese Patent Publication No. 502891/1987 (corresponding to WO 86/05694) discloses a composition for promoting the absorption of an orally administered substance into the body which comprises (a) at least one fatty acid having 14 to 18 carbon atoms, (b) at least one monoglyceride of glycerol and a fatty acid having 14 to 18 carbon atoms and (c) lysophosphatidylcholine containing a fatty acid component having 14 to 18 carbon atoms, optionally together with (d) a drug. Examples of the fatty acids constituting the components (a), (b) and (c) include palmitoleic acid, oleic acid, linoleic acid, linolenic acid and saturated fatty acids having 14 to 18 carbon atoms.

However it is the major object of said composition not to achieve a surface activity but to distribute an orally administered substance. Thus no composition falling within the range specified in the above Patent Publication can give a sufficiently intense activity. For example, a composition comprising said component (a) containing a saturated fatty acid having 14 to 18 carbon atoms shows extremely poor surface-active properties such as wettability and permeability.

It is an object of the present invention to provide a glycerophospholipid composition having a high safety and excellent surface-active properties including permeability, wettability and spreadability.

The present inventors previously found out that a composition comprising glycerophospholipid(s) partially hydrolyzed with phospholipase A-2, unsaturated fatty acid monoglyceride(s) and/or medium-chain fatty acid monoglyceride(s) has a high safety and intense surface-active properties and can be economically produced. Thus they have applied for a patent of such a composition (cf. Japanese Patent Laid-Open No. 207093/1987 and 240205/1987).

Furthermore, the present inventors found out that a composition comprising (1) partially deacylated glycerophospholipid(s) and (2) unsaturated fatty acid monoglyceride(s) and/or medium-chain fatty acid monoglyceride(s) and/or medium-chain fatty acid(s) has further enhanced surface-active properties and thus applied for a patent thereof (cf. Japanese Patent Laid-Open No. 106171/1988, No. 138150/1988 and No. 138151/1988).

Although the surface activity of these compositions described in the above patents would increase with an increase in the total amount of the monoglyceride(s) and fatty acid(s) to be added to the partially deacylated phospholipid(s), it is often required to use a solubilizer such as a bile acid salt.

The present inventors have further conducted extensive studies in order to improve the surface-active properties of glycerophospholipids. As a result, they have found out that the surface-active properties of partially deacylated glycerophospholipids can be readily enhanced by adding a definite amount of at least one saturated fatty acid having 8 to less than 14 carbon atoms to partially deacylated phosphatides (lysophosphatides).

The present invention, which has been completed based on the above finding, provides a glycerophospholipid composition having enhanced surface-active properties, obtainable by adding at least one saturated fatty acid having 8 to less than 14 carbon atoms in a total amount of 0.5 to 2 mol per mol of lysophosphatide(s),
with the proviso that the glycerophospholipid composition does not contain monoglyceride.

In the glycerophospholipid composition of the present invention, the surface-active properties of lysophospholipids, which are useful as a glycerophospholipid surfactant, are remarkably enhanced. Thus said glycerophospholipid composition has a high safety as well as excellent surface-active properties including permeability, wettability and spreadability.

The lysophosphatide which is one of the essential components of the glycerophospholipid composition of the present invention may be obtained by, for example, hydrolyzing glycerophospholipids such as soybean phospholipid, rapeseed phospholipid, corn germ phospholipid or yolk phospholipid with phospholipase A₂ or A₁ originating from pancreas, snake venom or microorganisms. Alternately, those obtained by partially hydrolyzing said materials with an acid such as sulfuric or phosphoric acid or chemically synthesized ones may be used therefor. When a chemically synthesized product is to be used, one or more saturated and/or unsaturated fatty acids having 12 to 22 carbon atoms may be selected as the fatty acid(s) constituting the lysophosphatide.

Although said lysophosphatides preferably comprises lysophosphatidylcholine, they may include lysophospholipids containing, for example, ethanolamine, inositol, serine or glycerol, in addition to the lysophosphatidylcholine. Furthermore, they may contain a small amount of glycolipid(s) or phosphatidic acid. These lysophosphatides may contain diacylphosphatides. In this case, it is preferable that the ratio by weight of the monoacylphosphatides (lysophosphatides) to the diacylphosphatides is at least 50/50. When this ratio is smaller than 50/50, satisfactory surface-active properties can be hardly achieved.

These lysophosphatides may be analyzed by various methods. For example, they may be developed with thin layer chromatography and each spot thus obtained is then analyzed. Alternately, they may be analyzed by using a TLC-FID analyzer (Iatroscan method), by high bperformance liquid chromatography (HPLC) or by combining these procedures.

Examples of saturated fatty acids having 8 to less than 14 carbon atoms, which are one of the essential components of the composition of the present invention, include caprylic, capric and lauric acid. These medium-chain fatty acids may contain a relatively small amount of long-chain fatty acid(s). Either one of the above-mentioned medium-chain fatty acids or a mixture thereof may be used. Furthermore, mixed fatty acids obtained by hydrolyzing, for example, coconut oil or palm kernel oil or those originating from animal milk fats may be employed therefor.

In the glycerophospholipid composition of the present invention, at least one fatty acid selected from the group consisting of said saturated fatty acids having 8 to less than 14 carbon atoms is added to said lysophosphatide(s) at a ratio of 0.5 to 2 mol (in total), preferably 0.75 to 1.5 mol, per mol of the lysophosphatide(s) in terms of pure matters. When mixed fatty acids are to be used, the amount of the same to be added is calculated based on the average molecular weight thereof. When the ratio of the fatty acid(s) is smaller than 0.5 mol per mol of the lysophosphatide(s), the obtained composition shows unsatisfactory surface-active properties. When more than 2 mol of the fatty acid(s) are added per mol of the lysophosphatide(s), on the other hand, the surface-active properties of the obtained composition are not enhanced any longer and, furthermore, the solubility of the composition in water is lowered in this case. The addition of the above-mentioned saturated fatty acid(s) having 8 to less than 14 carbon atoms at a ratio of 2 mol per mol of the lysophosphatide(s) or more would never deteriorate the effectiveness.

In the case of a preferable example of the glycerophospholipid composition of the present invention, the lysophosphatide comprises lysophosphatidylcholine or phosphatides rich in lysophosphatidylcholine. When the lysophosphatide contains lipids other than choline lipids, said lipids are substantially lysolipids.

In the glycerophospholipid composition of the present invention, it becomes more difficult to achieve elevated surface-active properties as the content of diacyl phospholipid(s) increases. When said saturated fatty acid(s) having 8 to less than 14 carbon atoms are to be added to said lysophosphatide(s), lauric acid carrying 12 carbon atoms shows the highest effect of enhancing the surface-active properties, from the viewpoints of, for example, permeability and spreadability.

The term "surface-active properties" used herein means activities including, for example, permeability, spreadability and lowering surface tension. These activities are compared with each other by using aqueous solutions of compositions to be tested, which compositions have the same total solid contents (expressed in % by weight) or the same molar concentration of the total solid matters, regardless of the differences in composition. The glycerophospholipid composition of the present invention shows remarkably enhanced activities, compared with the starting lysophosphatide(s) or those containing diacylphosphatide(s) employed alone.

The glycerophospholipid composition of the present invention may be prepared by, for example, the following methods.
(1) Lysophosphatide(s) optionally containing diacylphosphatide(s) are mixed with fatty acid(s) under reduced pressure or under a nitrogen atmosphere by heating to thereby give the glycerophospholipid composition of the present invention in the form of a paste.
(2) Lysophosphatide(s) optionally containing diacylphosphatide(s) and fatty acid(s) are dissolved in a solvent such as an alcohol or hexane. Next the solvent is removed under reduced pressure to thereby give the glycerophospholipid composition of the present invention.
(3) Lysophosphatide(s) optionally containing diacylphosphatide(s) and fatty acid(s) are dissolved in water to thereby give the glycerophospholipid composition of the present invention in the form of an aqueous solution or an aqueous paste.
(4) Glycerophospholipid(s) are dispersed in water containing a small amount of calcium ions. The obtained dispersion is then treated with phospholipase A₂ originating from, for example, pancreas to thereby give a mixture of lysophosphatides and fatty acids. After thermally inactivating the enzyme, the glycerophospholipid composition of the present invention is obtained.

The glycerophospholipid composition of the present invention thus obtained shows considerably enhanced surface-active properties, compared with not only those of the starting diacylphosphatides but also those of the corresponding lysophosphatide(s). Furthermore, the preferable glycerophospholipid composition described above show remarkably enhanced surface-active properties.

The above-mentioned lysophosphatides may be obtained by various methods described in, for example, Japanese Patent Laid-Open No. 44893/1988 and No. 279753/1988. Furthermore, the lysophosphatide mixtures thus obtained may be treated with a solvent such as an aqueous alcohol so as to elevate the content of lysophosphatidylcholine or subjected to, for example, silica gel chromatography so as to elevate the purity of lysophosphatidylcholine. The resulting products are also available in the present invention. As the above-mentioned fatty acids, mixed fatty acids originating from natural fats and oils may be used as such. Alternately they may be optionally converted into the corresponding methyl esters and then fractionated by distillation.

The glycerophospholipid composition of the present invention is useful as a permeating agent, a wetting agent or a spreading agent. Furthermore, it is available in dispersing fine particles, emulsifying agent and solubilizing agent. An optimum composition suitable for the aimed purpose may be obtained by appropriately selecting the phospholipids, the degree of their decomposition and the type and amount of the fatty acids to be added.

More particularly, the glycerophospholipid composition of the present invention may be used as a penetrating and wetting agent in order to, for example, improve the wetting properties of powdery foods, feeds, cosmetics and drugs. Further, it may be used as a spreading agent for hair rinses and chemicals for plants and insects. Furthermore, it may be used for improving the wetting of fiber products such as paper and fabric. Thus the glycerophospholipid composition of the present invention may be applied to, for example, oil-in-water and water-in-oil emulsification of oily materials such as edible fats and oils, vegetable essential oils and paraffins; dispersion, wetting in water or acceleration of dispersion of various powders such as cocoa powder, powdery instant foods, powdery spices, mildew-proofing agents such as butyl p-hydroxy-benzoate and various pigments; emulsification or dispersion of the above-mentioned materials at a certain high salt concentration or under acidic conditions; solubilization of hydrophilic materials in fats or oils; and solubilization of hydrophobic substances in water.

Furthermore the glycerophospholipid composition of the present invention solubilize proteins such as enzymes from animal or vegetable cell membranes. Although polyoxyethylenenonylphenolether has been frequently used for this purpose as an intense surfactant, it would sometimes denature some proteins. Lysophosphatidylcholine is also available therefor. However the effect of this compound is not always satisfactory. In contrast to these conventional surfactants, the composition of the present invention shows a high safety as well as an intense activity.

In addition, the high permeability of the composition of the present invention makes it useful in promoting the absorption of drugs through intestine, skin or mucosa. Furthermore, some physiologically active substances such as polyunsaturated fatty acids, e.g., eicosapentaenoic acid or γ-linolenic acid or odd-number acids such as pentadecanoic acid may be introduced into the composition of the present invention so as to use the composition of the present invention per se as a drug. Further, the composition of the present invention may be used in a fat emulsion together with other nutrients so as to give a highly digestible intestinal nutrient preparation. The effect on promoting the absorption of drugs with the composition of the present invention can be widely applied not only to mammals but also to other animals such as insects as well as plants such as crops.

The composition of the present invention is soluble both in water and in nonpolar solvents such as oils and forms a micell therein. Thus it is amphipathic.

To further illustrate the present invention, the following Examples, Comparative Examples and Referential Examples will be given, wherein all percentages and parts are by weight, unless otherwise noted.

The following fatty acids were employed in the following Examples and Comparative Examples wherein each fatty acid is represented in the abbreviation given in parentheses.
Lauric acid (C₁₂:₀FA)
   NAA-122, a product of Nippon Oil and Fats Co., Ltd.
   C₁₂ acid: 99.7%
Capric acid (C₁₀:₀FA)
   Lunac 10-95, a product of Kao Corporation
   C₁₀ acid: >95%.
Caprylic acid (C₈:₀FA)
   Lunac 8-95, a product of Kao Corporation
   C₈ acid: >95%.

In the following Examples and Comparative Examples, the surface-active properties of each sample were determined as follows.

### (1) Capability of lowering the surface tension:

The surface tension at 25°C was measured with a surface tension meter CBVP A-3 (mfd. by Kyowa Kagaku K.K.) provided with a platinum plate.

### (2) Canvas disc sedimentation:

The permeability at 25°C was measured according to a process described in "Kaimenkasseizai Binran", Sangyo Tosho K.K., p. 860 (1970) with the use of a piece (diameter: 2.54 cm) of thick canvas cloth. This is a modified method of Seyferths' (Am. Deystuff Reptr. 27, 525-532, 1938.)

### (3) Contact angle:

The contact angle on bees wax at 25°C was measured with a contact angle meter Elmer 13 (mfd. by Elmer K.K.).

### (4) Foliar-spreading:

A spreading test was conducted in the following manner by using an aqueous solution containing each composition having a solid content of 0.2 % by weight, 0.06% by volume of xanthan gum (Echogum, a product of Merck) and a small amount of an edible blue colorant No. 1 (Brilliant Blue FCF, a product of Sanei Kagaku Kogyo K.K.) as a spreading solution.

A test piece (10 x 6 cm) was prepared from the sixth to eighth leaf, counting from outside, of a marketed cabbage. The test piece was immersed in 300 ml of the spreading solution at 25°C for 10 seconds and then dried on a filter paper. Thus the spreading of the blue colorant was observed.

The evaluation was conducted based on the following criteria:
5: more than 90% of the foliar surface was colored blue,
4: 70 to 90% thereof was colored blue,
3: 50 to 70% thereof was colored blue,
2: 30 to 50% thereof was colored blue, and
1: 10 to 30% thereof was colored blue.

### Referential Example 1

Commercial soya lecithin (a product of Ajinomoto Co.) were defatted with acetone and fractionated with an aqueous alcohol to thereby give phosphatides containing 70% of phosphatidylcholine. The obtained product was treated with phospholipase A₂ (Lecithase 10-L, a product of Novo) and treated with acetone to thereby remove fatty acids. After further treating with an aqueous alcohol and subjecting to silica gel column chromatography with the use of an aqueous alcohol, phosphatides containing 98% or more of lysophosphatidylcholine were obtained. These phosphatides will be called Soybean LPC 98 hereinafter.

### Referential Example 2

14 parts of water was added to 100 parts of commercial soya lecithin (a product of Ajinomoto Co.). Further, 0.25 part of phospholipase A₂ (Lecithase 10-L, a product of NOvo) was added thereto. Then the obtained mixture was stirred and allowed to stand at 50 to 55°C for 15 and 36 hours so as to conduct monoacylation. Each reaction mixture was dehydrated under reduced pressure and centrifuged to thereby remove triglycerides and most of fatty acids. Next, it was treated with acetone to thereby remove residual impurities. The insoluble matters were dried under reduced pressure. Thus lysophosphatides (1) and (2) containing diacylphosphatides were obtained.

The contents of monoacylphosphatides in the lysophosphatides (1) and (2) thus obtained based on the total amounts of mono- and diacylphosphatides were 66% and 82% respectively.

These lysophosphatides (1) and (2) will be called Soybean LP 66 and Soybean LP 82 respectively.

### Referential Example 3

The Soybean LP 82 obtained in Referential Example 1 was dissolved in aqueous ethanol and cooled to thereby give phosphatides (3) containing 57% of lysophosphatidylcholine and 80% of total lysophosphatides. The phosphatides (3) will be called Soybean LPC 57 hereinafter.

### Referential Example 4

Marketed yolk phospholipids (Yolk Lecithin, a product of Asahi Chemical Industry Co., Ltd., purity: 96%) were ultrasonically dispersed in water and treated with phospholipase A₂ for 40 hours in the same manner as the one described in Referential Example 1. After removing residual impurities by treating with acetone, yolk phosphatides (4) were obtained.

The phosphatides (4) contained 86% by weight of monoacylphosphatides, based on the total amount of the mono- and diacylphosphatides and 65% of lysophosphatidylcholine. The phosphatides (4) will be called Yolk LP 86 hereinafter.

### Example 1

One mol of each fatty acid specified in Table 1 was added per mol of Soybean LPC 98 obtained in Referential Example 1 and aqueous solutions of final solid contents of 0.2% by weight and 0.5% by weight were prepared with the use of an ultrasonic wave generator. Then the surface-active properties of each aqueous solution were examined. Table 1 summarizes the results.

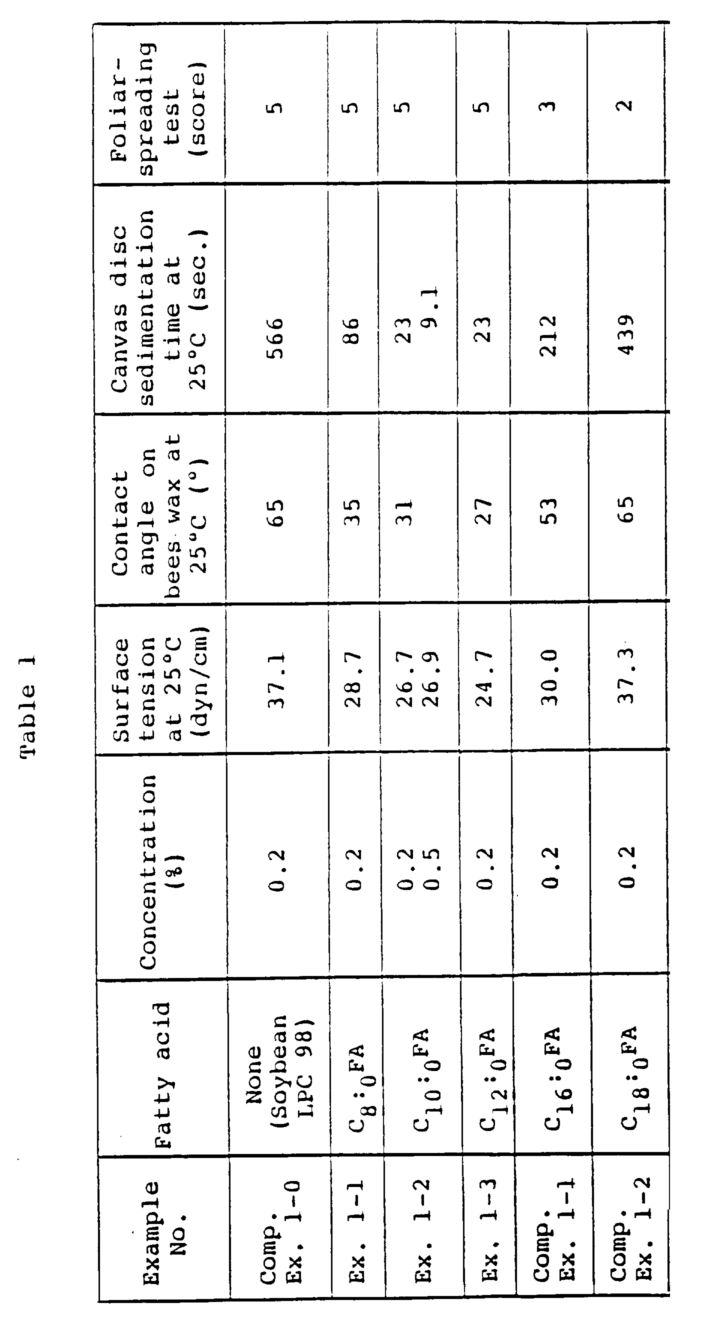

### Example 2

0.75 mol of Soybean LPC 98 was mixed with 0.25 mol of soybean phosphatidylcholine of a purity of 98% (Epicuron 200S, a product of Lucas Meyer, West Germany) to thereby give phosphatides containing 67% by weight of lysophosphatidylcholine which will be called LPC 67 hereinafter. To one mol of these phosphatides, was added 0.75 mol of each fatty acid specified in Table 2 and a 0.2% aqueous solution was prepared in the same manner as the one described in Example 1. The surface-active properties of each aqueous solution thus obtained were determined by the above-mentioned methods. The results are shown in Table 2 wherein a 0.2% aqueous solution of a composition having a ratio of monoacylphosphatides to diacylphosphatides of 3/1 is shown as Comp. Ex. 2-0.

**Table 2**

| Example No. | Fatty acid | Surface tension at 25°C (dyn/cm) | Contact angle on bees wax at 25°C (°C) | Canvas disc sedimentation time at 25°C (sec.) |
|---|---|---|---|---|
| Ex. 2-1 | C₁₀:₀FA | 28.5 | 38 | 69 |
| Ex. 2-2 | C₁₂:₀FA | 27.0 | 36 | 66 |
| Comp. Ex. 2-0 | None (Soybean LPC 67) | 41.2 | 53 | 684 |
| Comp. Ex. 2-1 | C₁₈:₀FA | 40.1 | 62 | 1071 |

### Example 3

To Soybean LPC 57 containing 80% of lysophosphatides obtained in Referential Example 3, was added each fatty acid specified in Table 3. After dissolving the obtained mixture in a small amount of a mixture of an alcohol and hexane (1 : 1), the solvent was removed under reduced pressure. Then the residue was dissolved in water to thereby give an aqueous solution of a solid content of 0.3%. The molar ratio of the lysophosphatides to the fatty acid was 1/0.7. The surface-active properties of each aqueous solution thus obtained were determined by the above-mentioned methods. Table 3 summarizes the results.

**Table 3**

| Example No. | Fatty acid | Surface tension at 25°C (dyn/cm) | Contact angle on bees wax at 25°C (°) | Canvas disc sedimentation time at 25°C (sec.) | Foliar-spreading test (score) |
|---|---|---|---|---|---|
| Ex. 3-1 | C₁₀:₀FA | 27.9 | 40 | 94 | 4 |
| Ex. 3-2 | C₁₂:₀FA | 26.7 | 37 | 78 | 4 |
| Comp. Ex. 3-1 | C₁₈:₀FA | 31.1 | 56 | 1130 | 2 |

### Example 4

The procedure of Example 1-2 was repeated except that the molar ratio of Soybean LPC 98 to a fatty acid was adjusted to 1/0.5 (Example 4-1), 1/1.5 (Example 4-2) and 1/2 (Example 4-3) while the total solid content was adjusted to 0.2% to thereby give an aqueous solution in each case. Then the surface-active properties of each aqueous solution were determined in the above-mentioned methods. The results as well as those of Example 1-2 are shown in Table 4.

**Table 4**

| Example No. | Molar ratio of Soybean LPC 98 to fatty acid/Fatty acid | Surface tension at 25°C (dyn/cm) | Contact angle on bees wax at 25°C (°) | Canvas disc sedimentation time at 25°C (sec.) |
|---|---|---|---|---|
| Ex. 4-1 | 1/0.5 | 28.3 | 46 | 113 |
| | C₁₀:₀FA | | | |
| Ex. 1-2 | 1/1 | 26.7 | 31 | 23 |
| | C₁₀:₀FA | | | |
| Ex. 4-2 | 1/1.5 | 26.1 | 29 | 23 |
| | C₁₀:₀FA | | | |
| Ex. 4-3 | 1/2 | 25.9 | 28 | 21 |
| | C₁₀:₀FA | | | |

### Example 5

The procedure of Example 3-1 was repeated except that the molar ratio of Soybean LPC 57 to a fatty acid was adjusted to 1/1.05 (Example 5-1) and 1/1.4 (Example 5-2) while the total solid content was adjusted to 0.3% to thereby give an aqueous solution in each case. Then the surface-active properties of each aqueous solution were determined in the above-mentioned methods. The results as well as those of Example 3-1 are shown in Table 5.

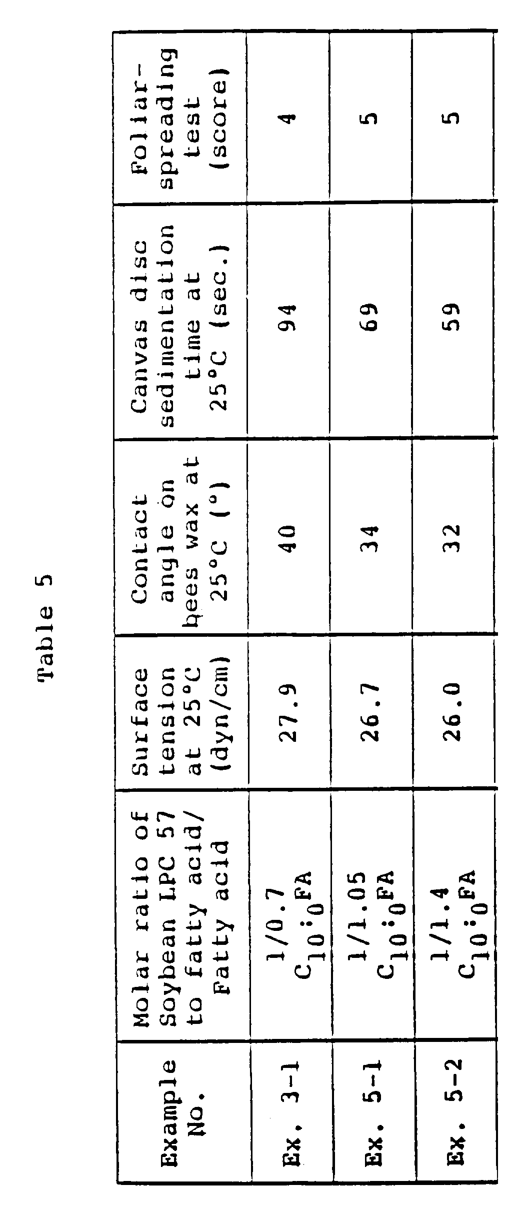

### Example 6

An aqueous solution containing 0.2% of Soybean LP 82 and 0.06% of lauric acid (Ex. 6-1) was ultrasonically prepared. The aqueous solution contained 3 mmol of the lysophosphatide and fatty acid. The surface-active properties of the aqueous solution was determined by the above-mentioned methods with the use of a 0.2% aqueous solution of Slybean LP 82 (Comparative Example 6-0) as a control. Table 6 shows the results.

**Table 6**

| Example No. | Fatty acid | Surface tension at 25°C (dyn/cm) | Contact angle on bees wax at 25°C (°) | Canvas disc sedimentation time at 25°C (sec.) | Foliar-spreading test (score) |
|---|---|---|---|---|---|
| Comp. Ex. 6-0 | None | 33.1 | 56 | 791 | 3 |
| Ex. 6-1 | C₁₂:₀FA | 25.9 | 38 | 124 | 5 |

### Example 7

According to the procedure described in Example 6, aqueous solutions each containing 0.25% (3 mmol) of Soybean LP 66 and 3 mmol of each fatty acid specified in Table 7 were prepared. The surface-active properties of each aqueous solution thus obtained were determined by the above-mentioned methods. Table 7 shows the results.

**Table 7**

| Example No. | Fatty acid | Surface tension at 25°C (dyn/cm) | Contact angle on bees wax at 25°C (°) | Canvas disc sedimentation time at 25°C (sec.) | Foliar-spreading test (score) |
|---|---|---|---|---|---|
| Comp. Ex. 7-0 | None | 33.3 | 59 | 627 | 2 |
| Ex. 7-1 | C₁₀:₀FA | 27.4 | 44 | 147 | 5 |
| Ex. 7-2 | C₁₂:₀FA | 26.0 | 39 | 92 | 5 |

### Example 8

NAA-122 (lauric acid), a product of Nippon Oil and Fats Co., Ltd., was added to 3 mmol of egg lysolecithin-100 containing more than 99 % of egg lysolecithin, a product of Nippon Shoji Kaisha Ltd. to thereby give a solution containing 3 mmol of lauric acid (Example 8-1), according to the procedure described in Example 1. Then the surface-active properties of the solution thus obtained was determined by the above-mentioned methods. Table 8 shows the results. A solution containing 3 mmol of egg lysolecithin (Comparative Example 8-0) was used as a control.

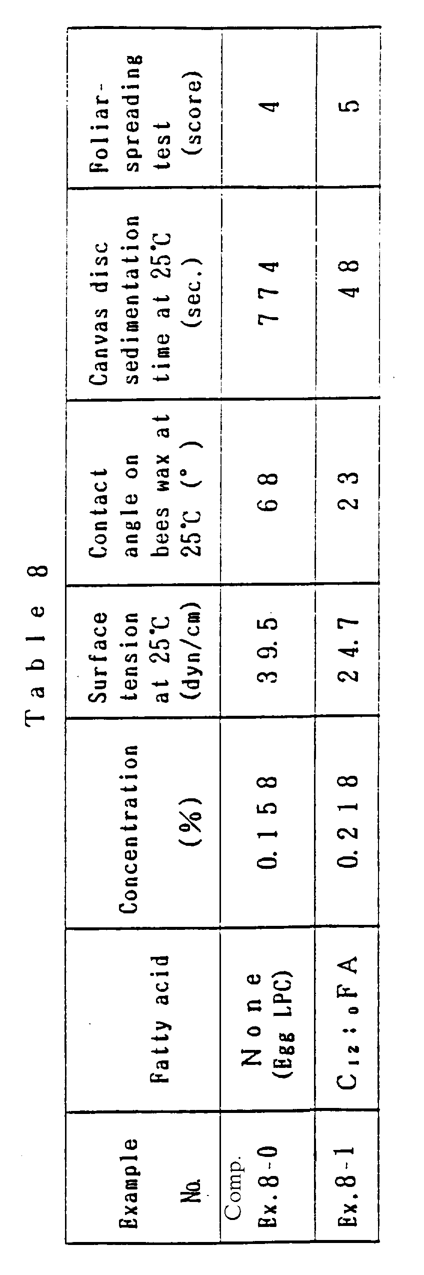

## Claims

1. A glycerophospholipid composition having enhanced surface-active properties, obtainable by adding at least one saturated fatty acid having 8 to less than 14 carbon atoms in a total amount of 0.5 to 2 mol per mol of lysophosphatide(s),
with the proviso that the glycerophospholipid composition does not contain monoglyceride.

2. The glycerophospholipid composition of claim 1 further comprising diacylphosphatide(s), wherein the ratio by weight of lysophosphatide(s) to diacylphosphatide(s) is 50/50 or more.

3. The glycerophospholipid composition of claim 1, wherein said lysophosphatide is lysophosphatidylcholine.

## Patentansprüche

1. Glycerophospholipidzusammensetzung mit verbesserten oberflächenaktiven Eigenschaften, erhältlich durch Zugabe mindestens einer gesättigten Fettsäure mit 8 bis weniger als 14 Kohlenstoffatomen in einer Gesamtmenge von 0,5 bis 2 Mol pro Mol Lysophosphatid(en), mit der Maßgabe, daß die Glycerophospholipidzusammensetzung kein Monoglycerid enthält.

2. Glycerophospholipidzusammensetzung nach Anspruch 1, weiterhin umfassend Diacylphosphatid(e), worin das Gewichtsverhältnis von Lysophosphatid(en) zu Diacylphosphatid(en) 50/50 oder mehr beträgt.

3. Glycerophospholipidzusammensetzung nach Anspruch 1, worin das Lysophosphatid Lysophosphatidylcholin ist.

## Revendications

1. Composition à base de glycérophospholipides ayant des propriétés tensio-actives renforcées, pouvant être obtenue par addition d'au moins un acide gras saturé comportant de 8 à moins de 14 atomes de carbone, selon une quantité totale de 0,5 à 2 moles par mole d'un ou plusieurs lysophosphatides, à condition que la composition à base de glycérophospholipides ne contienne pas de monoglycéride.

2. Composition à base de glycérophospholipides selon la revendication 1, comprenant en outre un ou plusieurs diacylphosphatides, le rapport pondéral du ou des lysophosphatides au ou aux diacylphosphatides étant de 50/50 ou plus.

3. Composition à base de glycérophospholipides selon la revendication 1, dans laquelle le lysophosphatide est la lysophosphatidylcholine.
